# EUROPEAN PATENT APPLICATION

(11) **EP 4 035 698 A1**
(43) Date of publication of application: **03.08.2022**
(21) Application number: 21154039.8
(22) Date of filing: 28.01.2021
(51) Int. Cl.: A61L 15/26, A61L 15/42, A61L 15/44, H01G 11/48

(54) **WOUND DRESSING AND METHOD FOR PREPARING THE SAME**

(71) Applicant: Max-Planck-Gesellschaft zur Förderung der Wissenschaften e.V., 80539 München (DE)
(72) Inventor: WEIL, Tanja, 55124 Mainz (DE); TOMMASO, Marchesi D'Alvine, 55131 Mainz (DE); HERBERGER, Tilmann, 55118 Mainz (DE); HARVEY, Sean, 55128 Mainz (DE); SYNATSCHKE, Christopher, 65187 Wiesbaden (DE); ROSENAU, Frank, 73066 Uhingen (DE)
(74) Representative: v. Bezold & Partner Patentanwälte - PartG mbB

(57) **Abstract**

The invention relates to a wound dressing comprising at least the following components:
a substrate, a layer of a biocompatible polymer film provided on said substrate, wherein said biocompatible polymer film is electro-conductive and capable to scavenge reactive oxygen species, and one or more therapeutic agents adhering to or incorporated into said layer of a biocompatible polymer film.

In one preferred embodiment, said biocompatible polymer is a polydopamine/polypyrrol copolymer.

A further aspect of the invention relates to methods for preparing such a wound dressing.

In one specific embodiment, a method for preparing said wound dressing comprises at least the following steps:
a) preparing a biocompatible polymer film, which biocompatible polymer film is electro-conductive and capable to scavenge reactive oxygen species, by electropolymerisation on a conductive primary substrate;
b) transferring the biocompatible polymer film prepared in step a) onto a non-conductive substrate;
c) contacting the biocompatible polymer film or the composite material obtained in step a) or step b) with one or more therapeutic agents and immobilizing said therapeutic agent(s) to the polymer film or incorporating said therapeutic agent(s) into the layer of biocompatible polymer film provided on a substrate by means of covalent or non-covalent interactions between said therapeutic agent(s) and said biocompatible polymer film.

In an alternative embodiment, the biocompatible polymer film is prepared directly on a non-conductive substrate.

## Description

### Background of the invention

The present invention provides improved wound dressing materials with antioxidative, electroactive, and antibiotic properties as well as methods for preparing the same.

Current wound dressings, in particular skin wound dressings, are designed to possess the required structural and mechanical properties (such as air permeability, flexibility etc.) and, in order to provide a desired biological activity, often therapeutic agents such as biocides, anti-inflammatory compounds, growth factors etc. are incorporated.

However, they generally lack the ability to respond to physiological signals (i.e. electrical signals). Small direct current electrical signals have been detected in biological systems and these signals have been found to play an important role in directing cell migration in wound healing (e.g. Tang et al., ACS Appl. Mater. Interfaces 2019, 11, 7703-7714).

Also, oxidative damage by reactive oxygen species (ROS) in wounds is known to retard the healing process and polydopamine, a biocompatible, biodegradable polymer has been used as an antioxidant together with polypyrrol (an electroconductive polymer) in the form of PPY-PDA-nanoparticles in a ROS-scavenging, electroactive and osteoactive scaffold for bone regeneration (Zhou et al., Small 2019, 15, 1805440). However, the films and scaffold compositions described therein are not generally suitable for wound dressing purposes, in particular not for skin wound dressings. Kim et al. (ACS Appl. Mater. Interfaces 2018, 10, 33032-33042) describe the coating of electrodes with a copolymer from PDA and PPY and show favorable properties for growing nerve cells on this material as well as the use of such electrodes for measuring electrical signals in an animal model.

Dhand et al., Biomaterials 138 (2017), 153-168) disclose wound dressings based on nanofiber gelatin mats loaded with polyhydroxy antibiotics and crosslinked with polydopamine. Xuan et al. (Biomaterials 252 (2020), 120018) describe bilayered composite nanosheets which comprise a first layer of haemostatic and antimicrobial gelatin modified with dopamine, antimicrobial peptide (AMP) and Ca²⁺ cations (acting as coagulation factors) and a second supportive layer of polycaprolactone for sealing and healing soft tissue bleeding wounds. Zhang et al., Biomater. Sci., 2019, 7, 5232-5237, disclose that the polydopamine modification of silk fibroin membranes significantly promotes their wound healing effect. Further, Hong et al., Applied Surface Science 257 (2011), 6799-6803, disclose the preparation and antibacterial effects of dopamine-modified cotton fabrics with silver nanoparticles bound to striated polydopamine membranes. None of these polydopamine-containing materials and coatings exhibit electroconductive properties.

In view of this prior art, the main object underlying the present invention is the provision of improved wound dressing materials which overcome or considerably alleviate the drawbacks of the prior art in that they are widely applicable, in particular also for skin wound dressings, combine most or all of the above mentioned desirable characteristics and can be a manufactured in a relatively simple and cost-efficient manner.

This object is achieved according to the present invention by providing the wound dressing according to claim 1 and the method for preparing the same according to claims 8 and 13. Additional aspects and preferred embodiments of the invention are the subject of further claims.

### Description of the invention

The wound dressing according to the invention comprises at least the following components: a substrate, a layer of a biocompatible polymer film provided on said substrate, wherein said biocompatible polymer film is both electro-conductive and capable to scavenge reactive oxygen species, and one or more therapeutic agents adhering to or incorporated into said layer of a biocompatible polymer film.

In a preferred embodiment of the present invention, the wound dressing is a skin wound dressing.

The biocompatible polymer in said wound dressing may be any biocompatible polymer which is electroconductive and capable to scavenge reactive oxidative species (ROS), and which is also capable to bind or incorporate therapeutic agents to or into a continuous thin film of said polymer.

More specifically, in said wound dressing the biocompatible polymer is a homopolymer of one monomer or a statistical copolymer of at least 2 monomers which monomer(s) is/are selected from the group comprising dopamine, nitrodopamine, 6-fluorodopamine, D- or L-DOPA, norepinephrine, para-phenylenediamine, pyrrol, amino tyrosine.

In a preferred embodiment of the present invention, said biocompatible polymer is a copolymer, in particular a polydopamine/polypyrrol copolymer.

As already briefly mentioned above, polydopamine is an effective antioxidant with which reactive oxygen species can be scavenged to actively support the healing process, and polypyrrole represents an electroconductive and electroactive polymer, capable to transduce physiological signals. Both these favorable characteristics are also exhibited/maintained by the polydopamine/polypyrrole copolymers films prepared and used according to the present invention. Moreover, these films also exhibit desirable very high elastic moduli.

Typically, the biocompatible polymer film represents a thin film or ultrathin film having a thickness in the range from 2 nanometers to 100 micrometers, preferably from 5 nanometers to 5 micrometers or 5 nanometers to 500 nanometers. More preferably, the polymer film has a thickness in the range from 5 nanometers to 100 nanometers, in particular from 50 nanometers to 100 nanometers.

The possibility to provide these polymer films in nanoscopic dimensions in terms of thickness offers certain advantages when being applied as wound dressings:
1) Thin films allow for adaptation to wounds with irregular topologies:
   Regular bandaids or wound dressings will not adhere completely to tissues of irregular topologies and will buckle when applied to movable tissues, e.g. in the region of joints. In other words, the contact area between wound and dressing is not maximized. This is not the case for very thin films, because they can better adapt to irregular topologies and will show an intrinsic adhesive property due to interfacial interactions, leading to a complete coverage of non-smooth surfaces.
2) Thin films compared to regular bandaids are expected to have better permeability for nutrients, water and oxygen, which should improve wound healing.

The substrate used in the wound dressing of the invention is not especially limited and may be, e.g., any material commonly used in wound dressings of the prior art.

Typically, said substrate is selected from the group comprising fiber-based materials, including woven and non-woven fabrics, in particular tulle, films, gauze, hydrofiber-based fabrics, lint, fleece, hydrocolloids, hydrogels, films and foams.

More specifically, the material of said substrate may be also selected from the group comprising PU, nylon, cellulose, cotton, linen, polyethylene, alginate, silicone, silk, fibroin, polylactic-co-glycolic acid, polysiloxane, polyamides, polyacrylamide, peptides, chitosan, polypropylene, carbon, collagen, gelatin, pectin, polyesters, regenerated cellulose (rayon).

The therapeutic agent incorporated in the wound dressing of the invention is not especially limited and may be, e.g., any therapeutic agent which can bind or adhere to the biocompatible polymer films used in the wound dressing of the invention.

A variety of suitable therapeutic agents are known in the art and may be selected depending from the desired respective therapeutic effect, such as prevention or counteracting of infections, promotion of wound healing, enhancement of favorable immune reactions etc.

More specifically, the therapeutic agent may be selected from the group comprising anti-inflammatory molecules or immune-modulators, anti-oxidants, cytostatic agents, biocides, in particular anti-fungals, anti-bacterials, anti-virals, proteins, peptides, enzymes, antibodies, aptamers, growth factors or combinations thereof.

In one preferred embodiment, the therapeutic agent is a biocide, in particular an antimicrobial peptide (AMP). Antimicrobial peptides (AMPs) represent promising alternative agents against a plurality of multi-resistent pathogens, in particular fungi. AMPs reduce structural integrity of the cell walls of these pathogens by physical disintegration and/or pore formation and they are expected to provide treatment options even against organisms resistant to conventional antifungal drugs that typically address a distinct target structure (e.g. surface proteins, enzymes, etc.).

Especially preferred for use in the present invention is the AMP Cm-p5 derived from a peptide isolated from the coastal mollusk *Cenchritis muricatus.* This AMP is known to exhibit efficient antifungal activity against *C*. *albicans, C. parapsilosis, Cryptococcus neoformans* and *Trichophyton rubrum* with only negligible toxicity towards mammalian cells. Moreover, Cm-p5 is also effective against *Candida auris* infections, such as occurring in skin wounds after initial contamination.

A wound dressing according the present invention which comprises AMPs, in particular Cm-p5, represents a very effective means to deliver such peptides to the wound or site of infection in a safe and convenient fashion.

The therapeutic agent(s) may be bound to the biocompatible polymer film or incorporated into the layer of biocompatible polymer film provided on the substrate by means of covalent or non-covalent interactions between said therapeutic agent(s) and said biocompatible polymer film. These interactions may comprise any kind of covalent interaction, such as esterification, amide bond formation, Michael Addition reactions, imine formation, ether bonds etc., or non-covalent interactions, such as electrostatic binding, hydrophobic adsorption, hydrogen bonds, π-π interactions, metal complex formation or metal complex formation.

Principally, polymer films or coatings on substrates can be prepared by various methods using chemical or electrochemical approaches. For example, polydopamine coatings have been prepared by means of dip coating under basic conditions or electrochemically (Stöckle et al., Macromol. Symp. 2014, 346, 73-81).

Electropolymerization has several advantages compared to dip coating. It is a mild technique with which homogeneous and smooth films can be obtained. The film thickness can be finely controlled, even between 10 and 20 nm, and is permeable for water and small ions.

However, the electropolymerization has the drawback that the film needs to be prepared on a conductive substrate. Therefore, the present inventors developed a procedure with which a polymer film, in particular a polydopamine film or a polydopamine/polypyrrol copolymer film, can be successfully transferred on any substrate. Moreover, the present inventors also surprisingly found that therapeutic agents can be conveniently bound to or incorporated into said polymer films in the course of such polymerization and/or transfer processes.

Therefore, a second main aspect of the present invention relates to novel and especially favorable methods for preparing the wound dressing as described above.

Such method generally comprises at least the following steps:
a) preparing a biocompatible polymer film, which biocompatible polymer film is electro-conductive and capable to scavenge reactive oxygen species, by electropolymerisation on a conductive primary substrate;
b) transferring the biocompatible polymer film prepared in step a) onto a secondary, typically a non-conductive, substrate;
c) contacting the biocompatible polymer film or the composite material obtained in step a) or step b) with one or more therapeutic agents and immobilizing said therapeutic agent(s) to the polymer film or incorporating said therapeutic agent(s) into the layer of biocompatible polymer film provided on a substrate by means of covalent or non-covalent interactions between said therapeutic agent(s) and said biocompatible polymer film.

In a more specific embodiment of said method, step a) comprises the following steps:
- providing a reaction medium which comprises one or more monomers, an electrolyte, a solvent or a mixture of solvents, a conductive or semi-conductive substrate that is to be coated, and optionally a therapeutic agent,
- performing an electropolymerization reaction, preferably under conditions of controlled electron flow, for a suitable time period, preferably in the range from about 15 minutes to about 3 hours, to obtain a polymer film having a desired thickness,
- optionally removing the film-covered substrate from the reaction bath and rinsing to remove unbound monomers or oligomers.

Electropolymerization may be performed through one of several possible modes, i.e. potentio-dynamic, potentiostatic, galvanostatic, or galvanodynamic, where a flow of electrons induces a chemical reaction that leads to the crosslinking of the monomers in proximity of the substrate and subsequent film formation. Films of controllable thickness can be obtained by controlling the electron flow, e.g. by modulating the applied potential in cyclic voltammetry (potentio-dynamic mode).

The solvent in said electropolymerization reaction may be, e.g. selected from the group comprising water, dimethylsulfoxide, acetonitrile, ionic liquids or other solvents capable of dissolving electrolytes, or mixtures thereof.

The electrolyte may be for example para-toluene sulfonate, but other electrolytes, such as alkali metal chlorides, phosphates, sulfates etc., are also possible.

The conductive primary substrate is not especially limited and may be a metal or any kind of semiconductor. For example, gold-coated glass slides or indium-tin oxide glass slides, polypyrrole or a PEDOT:PSS ((poly(3,4-ethylenedioxythiophene)-polystyrene sulfonate) semiconductor have been successfully used by the inventors.

Typically, the electropolymerization reaction in step a) comprises a plurality of polymerization cycles, such as 5-15 polymerization cycles of each 200-500 seconds, which allows to adjust the final thickness of the resulting polymer film as desired.

In another specific embodiment of said method step b) comprises
- depositing a solution of a water-soluble polymer on top of the substrate-bound film and, after drying, mechanically stripping the composite of electropolymerized film and polymer from the conductive substrate and immersing the same in a solvent bath capable to dissolve the polymer and, after dissolution of the polymer, immersing the final non-conductive substrate into the solvent bath, depositing the polymer film on the non-conductive substrate and removing the composite of polymer film and non-conductive substrate from the solvent bath.

The water soluble polymer is not especially limited and principally may be any polymer (homopolymer or copolymer) that is water soluble and capable of attractive interactions with the electropolymerized film. Preferably, the glass transition temperature of these polymers should be below 40 degrees centigrade so that sufficient flexibility of the polymer film during the transfer process is achieved. In particular, these water soluble polymers may be selected from the group comprising different types of cellulose derivatives, e.g. hydroxy propyl or hydroxyl methyl cellulose, polyethylene glycol, polyhydroxy ethyl methacrylate, and polyvinylalcohol-co-polyvinylacetate copolymers. In a more preferred embodiment, the water soluble polymer is a polyvinylalcohol-co-polyvinylacetate copolymer.

Preferably, the polyvinylalcohol-co-polyvinylacetate copolymer used in step b) has a ratio of about 80% PVA to about 20% PVAc.

In another specific embodiment of said method step c) comprises
- immersing the biocompatible polymer film or the composite material obtained in step a) or step b) in a solution of the therapeutic agent for adsorption or reaction with the film, or
- spraying the biocompatible polymer film or the composite material obtained in step a) or step b) with a solution containing the therapeutic agent and letting it dry.

The stripping technique as described above allows easy transfer of the electropolymerized films onto other substrates, but may also be used to directly apply the films to a wound site, since, e.g., PVA or PVA-PVAc will dissolve when coming in contact with water and/or bodily fluids. This is especially interesting when utilizing these films for closing wounds on internal organs.

An alternative method for preparing the wound dressing according to the present invention directly on a non-conductive substrate comprises at least the following steps:
a) preparing a biocompatible polymer film, which biocompatible polymer film is electro-conductive and capable to scavenge reactive oxygen species, by polymerisation on a non-conductive substrate;
b) contacting the biocompatible polymer film or the composite material obtained in step a) with one or more therapeutic agents and immobilizing said therapeutic agent(s) to the biocompatible polymer film or incorporating said therapeutic agent(s) into the layer of biocompatible polymer film provided on the substrate by means of covalent or non-covalent interactions between said therapeutic agent(s) and said biocompatible polymer film.

More specifically in this method step a) comprises:
- providing a reaction medium which comprises one or more monomers, a solvent or a mixture of solvents, a non-conductive substrate that is to be coated, and optionally a polymerization initiator and/or a therapeutic agent,
- performing a polymerization reaction, optionally under conditions of visible light or UV-irradiation,
   for a suitable time period, preferably in the range from 5 to 1200 minutes, to obtain a polymer film having a desired thickness,
- optionally removing the film-covered substrate from the reaction bath and rinsing to remove unbound monomers or oligomers.

The solvent in said electropolymerization reaction may be, e.g., selected from the group comprising water, dimethylsulfoxide, acetonitrile, ionic liquids or other solvents capable of dissolving electrolytes, or mixtures thereof.

The polymerization initiator for this kind of reaction is not especially limited and may be an acid or base, oxidative or reductive species. In one specific embodiment (photo-fenton reaction) a reagent comprising complexed Fe³⁺/H₂O₂ can be used as initiator. Also, standard UV initiators such as ACVA (4,4'- azobis(4-cyanopentanoic acid) are suitable.

It is further possible to conduct this reaction in the absence of any polymerization initiator.

In another specific embodiment of the above described methods according to the present invention step a) further comprises subjecting the obtained polymer film to a further crosslinking step in a second reaction medium by immersion in an aqueous buffer with pH > 7, preferably carbonate buffer (e.g. 100 mM), pH 8-10.

More specifically, in the above described methods according to the present invention the biocompatible polymer is a homopolymer of one monomer or a statistical copolymer of at least 2 monomers which monomer(s) is/are selected from the group comprising dopamine, nitrodopamine, 6-fluorodopamine, D- or L-DOPA, norepinephrine, para-phenylenediamine, pyrrol, amino tyrosine.

In a preferred embodiment of the present invention, said biocompatible polymer is a copolymer, in particular a polydopamine/polypyrrole copolymer.

Typically, the biocompatible polymer film used for preparing the wound dressing of the present invention is a thin film or ultrathin film having a thickness in the range from 2 nanometers to 100 micrometers, preferably from 5 nanometers to 5 micrometers or 5 nanometers to 500 nanometers, in particular 5 to 100 nanometers or 50 to 100 nanometers.

The substrate used for preparing the wound dressing of the present invention is not especially limited and may be, e.g., any material commonly used in wound dressings of the prior art.

Typically, said substrate is selected from the group comprising fiber-based materials, including woven and non-woven fabrics, in particular tulle, films, gauze, hydrofiber-based fabrics, lint, fleece, hydrocolloids, hydrogels, films and foams.

More specifically, the material of said substrate may be also selected from the group comprising PU, nylon, cellulose, cotton, linen, polyethylene, alginate, silicone, silk, fibroin, polylactic-co-glycolic acid, polysiloxane, polyamides, polyacrylamide, peptides, chitosan, polypropylene, carbon, collagen, gelatin, pectin, polyesters, regenerated cellulose (rayon).

### Brief Description of the Figures

**Fig. 1** shows schematically the main steps in the preparation of a wound dressing according to the invention.
**Fig. 2** shows schematically the preparation of a primary conductive substrate, i.e. a gold-coated glass slide.
**Fig. 3** shows schematically a device for depositing a polymer film on a primary conductive substrate (gold slide) by means of electropolymerization.
**Fig. 4** shows schematically the transfer of a polymer film on a membrane support by means of vacuum filtration.

The following Examples are provided to illustrate the present invention in more detail, however, without limiting the same to the specific conditions and parameters thereof.

### EXAMPLE 1

### Preparation of polydopamine or polydopamine/polypyrrol thin films

### Preparation of a primary conducting substrate (gold working electrode)

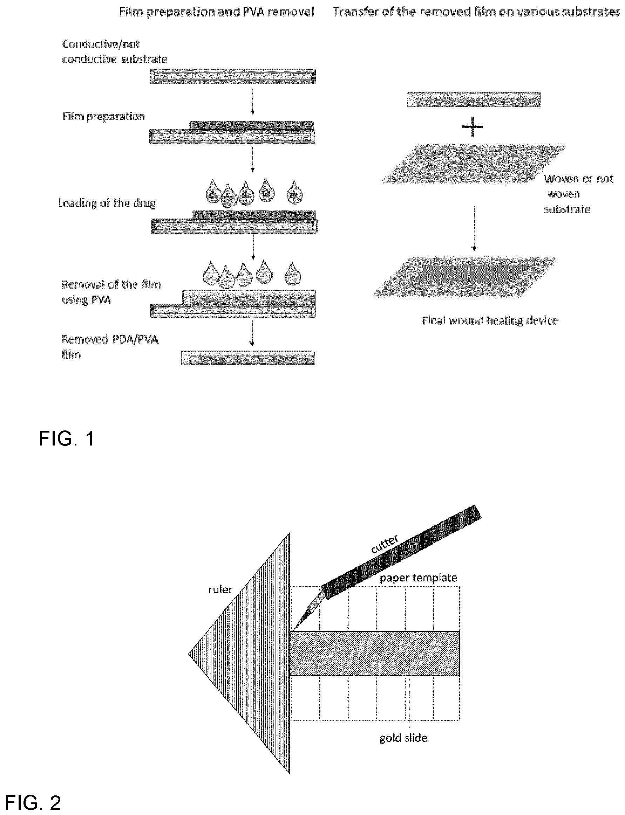

A gold-coated (1000 Å) glass slide is scratched with a diamond cutter (as depicted in Fig. 2). Water is dropped along the scratch and the slide is broken with glass pliers. The cut piece is dried with nitrogen and afterwards plasma cleaned for 10 minutes in argon plasma under a pressure of 6 mbar.

### Preparation of a starting solution (phosphate buffer + dopamine)

A 100 mM phosphate buffer at pH 7 is prepared using sodium phosphate dibasic (99%) and sodium phosphate monobasic (99%) in Milli-Q water. The phosphate buffer is deoxygenated via nitrogen purging for 15 minutes. Then, 1 mg/ml dopamine hydrochloride is added and the solution is continuously purged until all the solids are dissolved.

### Preparation of PDA polymer films using cyclic voltammetry

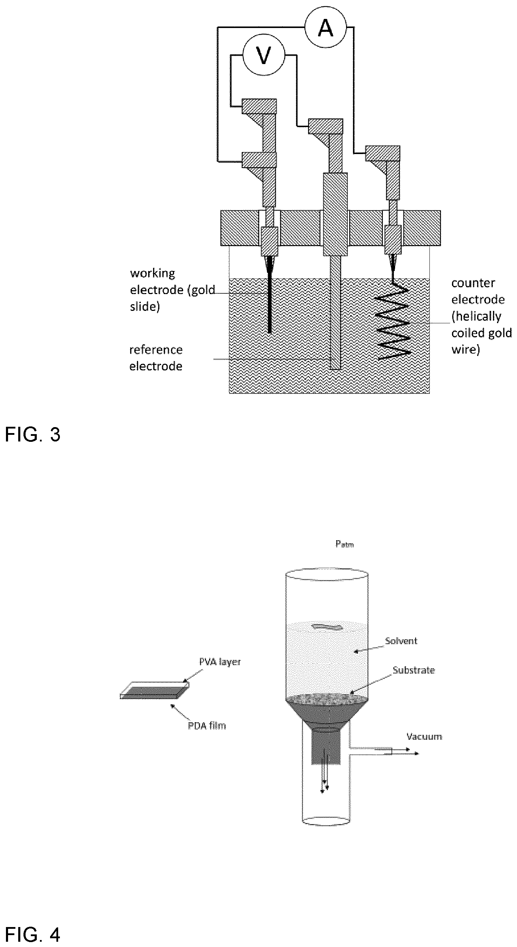

A three electrode system is set up as depicted in Fig. 3. The gold covered side of the glass slide, used as a working electrode, faces inward. A Ag/AgCl, 3M KCl electrode is used as the reference electrode and a helically coiled gold wire as the counter electrode. While the atmosphere can be air, inert atmosphere (e.g. N₂) results in a more homogeneous film morphology. The potential between reference and working electrode is swept between -0.5 V and +0.5 V with a rate of 10 mV/s for several cycles. A PDA film forms on the gold electrode. The film thickness can be adjusted via the number of cycles. In case of a copolymerization of PDA and PPY, the potential is swept between -0.65 V and +0.8 V at the same sweep rate.

### PDA-PPY copolymerization:

The procedure for PDA-PPy copolymerization follows the same steps as the PDA polymerization, only that the buffer contains pyrrole and p-toluenesulfonate as an electrolyte in addition to the previously used dopamine-hydrochloride. The components can be mixed in varying molarities, which results in variations regarding the thickness, the adhesiveness and the electrical conductivity of the film.

### EXAMPLE 2

### Transfer of polydopamine or polydopamine/polypyrrol thin films onto non-conductive substrates for wound dressings

Firstly, the PDA or PDA/PPY covered gold slide is immersed in carbonate buffer (sodium bicarbonate (>99.7%) and sodium carbonate (>99.8%)) at pH 10 for 30 min. This results in further crosslinking and subsequently a more stable film. In the next step, the slide is immersed in phosphate buffer and three additional cycles are applied over a potential range from -0.8 V to 1.2 V to overoxidize the system and thereby reduce the adhesion between the film and the gold substrate. Then the PDA (or PDA/PPY) film is coated with a sacrificial PVA-PVAc layer, which serves as a support for the transfer process. The coating is performed via drop casting of a PVA-PVAc solution (200 µL, 10 wt%) in water. After drying at 40 °C for approximately 25 min, the PDA/PVA-PVAc film is mechanically stripped off the substrate. For transfer to mesh-type substrates, resulting in free-standing films on a porous support, it is placed on the surface of a water bath with the PDA side facing the liquid. The PVA-PVAc layer expands and dissolves, leaving the bare PDA (or PDA/PPY) film floating on water. Then, the PDA (or PDA/PPY) film is removed from the bottom with a supporting structure and thereby transferred to a substrate.

As an alternative to the above approach, the PDA film (or PDA/PPY film) can be transferred to a porous substrate by applying a vacuum from below to remove the solution and deposit the film on the substrate. Then the sample is washed in water for 1 h and in 45 °C water for 1 h to completely remove the PVA-PVAc and subsequently dried at room temperature for 1 h. Alternatively, the PDA (PDA/PPY) may also be transferred in the dry state by directly placing the PDA/PVA-PVAc film on the new substrate and gently washing with water to remove PVA-PVAc.

## Claims

1. A wound dressing, in particular skin wound dressing, comprising at least the following components:
- a substrate
- a layer of a biocompatible polymer film provided on said substrate, wherein said biocompatible polymer film is electro-conductive and capable to scavenge reactive oxygen species
- one or more therapeutic agents adhering to or incorporated into said layer of a biocompatible polymer film.

2. The wound dressing according to claim 1, wherein said biocompatible polymer is a homopolymer of one monomer or a statistical copolymer of at least 2 monomers which monomer(s) is/are selected from the group comprising dopamine, nitrodopamine, 6-fluorodopamine, D- or L-DOPA, norepinephrine, para-phenylenediamine, pyrrol, amino tyrosine, and in particular a polydopamine/polypyrrol copolymer.

3. The wound dressing according to claim 1 or 2, wherein said biocompatible polymer film has a thickness in the range from 2 nanometers to 100 micrometers, preferably from 5 nanometers to 500 nanometers, in particular 10 nanometers to 100 nanometers.

4. The wound dressing according to any one of claims 1 to 3, wherein said substrate is selected from the group comprising fiber-based materials, including woven and non-woven fabrics, in particular tulle, films, gauze, hydrofiber-based fabrics, lint, fleece, hydrocolloids, hydrogels, films and foams.

5. The wound dressing according to claim 4, wherein the material of said substrate is selected from the group comprising PU, nylon, cellulose, cotton, linen, polyethylene, alginate, silicone, silk, fibroin, polylactic-co-glycolic acid, polysiloxane, polyamides, polyacrylamide, peptides, chitosan, polypropylene, carbon, collagen, gelatin, pectin, polyesters, regenerated cellulose (rayon).

6. The wound dressing according to any one of claims 1 to 5, wherein the therapeutic agent is selected from the group comprising anti-inflammatory molecules or immune-modulators, anti-oxidants, cytostatic agents, biocides, in particular anti-fungals, anti-bacterials, anti-virals, proteins, peptides, enzymes, antibodies, aptamers, growth factors or combinations thereof.

7. The wound dressing according to claim 6, wherein the therapeutic agent is an antimicrobial peptide, in particular Cm-p5.

8. A method for preparing the wound dressing according to any one of claims 1-7, comprising
at least the following steps:
a) preparing a biocompatible polymer film, which biocompatible polymer film is electro-conductive and capable to scavenge reactive oxygen species, by electropolymerisation on a conductive primary substrate;
b) transferring the biocompatible polymer film prepared in step a) onto a non-conductive substrate;
c) contacting the biocompatible polymer film or the composite material obtained in step a) or step b) with one or more therapeutic agents and immobilizing said therapeutic agent(s) to the polymer film or incorporating said therapeutic agent(s) into the layer of biocompatible polymer film provided on a substrate by means of covalent or non-covalent interactions between said therapeutic agent(s) and said biocompatible polymer film.

9. The method according to claim 8, wherein step a) comprises:
- providing a reaction medium which comprises one or more monomers, an electrolyte, a solvent or a mixture of solvents, a conductive or semi-conductive substrate that is to be coated, and optionally a therapeutic agent,
- performing an electropolymerization reaction, preferably under conditions of controlled electron flow, for a suitable time period, preferably in the range from about 15 minutes to about 3 hours, to obtain a polymer film having a desired thickness,
- optionally removing the film-covered substrate from the reaction bath and rinsing to remove unbound monomers or oligomers.

10. The method according to any one of claims 8 to 9, wherein the electropolymerization reaction in step a) comprises 5-15 polymerization cycles of each 200-500 seconds.

11. The method according to any one of claims 8 to 10, wherein step b) comprises
- depositing a solution of a water-soluble polymer on top of the substrate-bound film and, after drying, mechanically stripping the composite of electropolymerized film and polymer from the conductive substrate and immersing the same in a solvent bath capable to dissolve the polymer and, after dissolution of the polymer, immersing the final non-conductive substrate into the solvent bath, depositing the polymer film on the non-conductive substrate and removing the composite of polymer film and non-conductive substrate from the solvent bath.

12. The method according to any one of claims 8 to 11, wherein step c) comprises
- immersing the biocompatible polymer film or the composite material obtained in step a) or step b) in a solution of the therapeutic agent for adsorption or reaction with the film, or
- spraying the biocompatible polymer film or the composite material obtained in step a) or step b) with a solution containing the therapeutic agent and letting it dry.

13. A method for preparing the wound dressing according to any one of claims 1-7, comprising at least the following steps:
a) preparing a biocompatible polymer film, which biocompatible polymer film is electro-conductive and capable to scavenge reactive oxygen species, by polymerisation on a non-conductive substrate;
b) contacting the biocompatible polymer film or the composite material obtained in step a) with one or more therapeutic agents and immobilizing said therapeutic agent(s) to the biocompatible polymer film or incorporating said therapeutic agent(s) into the layer of biocompatible polymer film provided on the substrate by means of covalent or non-covalent interactions between said therapeutic agent(s) and said biocompatible polymer film.

14. The method according to claim 13, wherein step a) comprises:
- providing a reaction medium which comprises one or more monomers, a solvent or a mixture of, a non-conductive substrate that is to be coated, and optionally a polymerization initiator and/or a therapeutic agent,
- performing a polymerization reaction, optionally under conditions of UV-irradiation,
for a suitable time period, preferably in the range from 5 to 1200 minutes, to obtain a polymer film having a desired thickness,
- optionally removing the film-covered substrate from the reaction bath and rinsing to remove unbound monomers or oligomers.

15. The method according to any one of claims 8 to 14, wherein step a) further comprises
- subjecting the obtained polymer film to a further crosslinking step in a second reaction medium by immersion in an aqueous buffer with pH > 7, preferably carbonate buffer, pH 8-10.

16. The method according to any one of claims 8 to 15, wherein said non-conductive substrate is selected from the group comprising fiber-based materials, including woven and non-woven fabrics, in particular tulle, films, gauze, hydrofiber-based fabrics, lint, fleece, hydrocolloids, hydrogels, films and foams.

17. The wound dressing according to any one of claims 1 to 7 or the method according to any one of claims 8 to 16, wherein said biocompatible polymer is a polydopamine/polypyrrol copolymer.
